# EUROPEAN PATENT APPLICATION

(11) **EP 4 530 345 A1**
(43) Date of publication of application: **02.04.2025**
(21) Application number: 23201058.7
(22) Date of filing: 29.09.2023
(51) Int. Cl.: C12N 5/0783, A61K 35/17

(54) **METHOD FOR THE STIMULATION OF T CELLS WITH IL-4 AND TNF ALPHA**

(71) Applicant: Rheinische Friedrich-Wilhelms-Universität Bonn, 53113 Bonn (DE)
(72) Inventor: KURTS, Christian, 53127 Bonn (DE); BAUMGART, Ann-Kathrin, 53127 Bonn (DE)
(74) Representative: Klöckner, Christoph

(57) **Abstract**

The present invention relates to methods for stimulating T cells, T cells obtainable from the methods, pharmaceutical compositions comprising such T cells, and the T cells and the pharmaceutical compositions for use as a medicament and in in a method of treating cancer in a subject.

## Description

### Technical Field

The present invention relates to methods for stimulating T cells, T cells obtainable from the methods, pharmaceutical compositions comprising such T cells, and the T cells and the pharmaceutical compositions for use as a medicament and in a method of treating cancer in a subject.

### Technological Background

The concept and advantages of T cells with synthetic antigen-specific T cell receptors (CAR-T cells) are well known in the art and have become a focus of pharmaceutical research and development in recent years. CAR T cells are being used in individual medicine for the treatment of cancers such as acute lymphoblastic leukaemia and B cell lymphomas (Arzneimitteltherapie 2019;37:64-8 and Arzneimitteltherapie 2019;37:60-3). This involves isolating the patient's own T cells, genetically modifying them and reinfusing them into the patient. However, the success of this therapy has not yet extended to solid tumors (Wagner, Jessica, et al. "CAR T cell therapy for solid tumors: bright future or dark reality?" Molecular therapy 28.11 (2020): 2320-2339). Solid tumors pose a particular therapeutic challenge as they generate an immunosuppressive milieu that limits the survival and functionality of immune cells (Labani-Motlagh, Alireza, Mehrnoush Ashja-Mahdavi, and Angelica Loskog. "The tumor microenvironment: a milieu hindering and obstructing antitumor immune responses." Frontiers in immunology 11 (2020): 940).

The progress made in CAR T cell therapy in recent years indicates that the potential of CAR T cells has not yet been exhausted (Chen, Kun, et al. "Clinical investigations of CAR-T cell therapy for solid tumors." Frontiers in Immunology 13 (2022): 896685). Improving the survival and functionality of CAR T cells in the tumor microenvironment could thus enable the treatment of solid tumors.

The differentiation of T cells into effector cells that can actively fight tumor cells, and their phenotype with regard to their persistency and efficacy, can be modulated by cytokines (Mittrücker, Hans-Willi, Alexander Visekruna, and Magdalena Huber. "Heterogeneity in the differentiation and function of CD8+ T cells." Archivum immunologiae et therapiae experimentalis 62 (2014): 449-458; Cox, Maureen A., Laurie E. Harrington, and Allan J. Zajac. "Cytokines and the inception of CD8 T cell responses." Trends in immunology 32.4 (2011): 180-186). These cytokines can be added to the culture medium during the generation of CAR-T cells. To date, IL-2, or the combination of IL-7 and IL-15, have mainly been used for this purpose (Zhou, Jianxia, et al. "Chimeric antigen receptor T (CAR-T) cells expanded with IL-7/IL-15 mediate superior antitumor effects." Protein & cell 10.10 (2019): 764-769; Xu, Yang, et al. "Closely related T-memory stem cells correlate with in vivo expansion of CAR. CD19-T cells and are preserved by IL-7 and IL-15." Blood, The Journal of the American Society of Hematology 123.24 (2014): 3750-3759). Other cytokines that have already been studied in this context include IL-12, IL-18 and IL-21 (Bell, Matthew, and Stephen Gottschalk. "Engineered cytokine signaling to improve CAR T cell effector function." Frontiers in Immunology 12 (2021): 684642; Xu, Xiao-Jun, et al. "Multiparameter comparative analysis reveals differential impacts of various cytokines on CART cell phenotype and function ex vivo and in vivo." Oncotarget 7.50 (2016): 82354). However, these cytokines have not yet achieved a breakthrough in the treatment of solid tumors.

Therefore, there is a need for a cytokine combination which provides a suitable stimulus for improving the expansion, persistence, and functionality of CAR-T cells for tumor therapy, in particular of solid tumors.

Thus, it is an object of the present invention to provide a novel method for stimulating T cells that yield T cells with an improved persistence and reduced expression of inhibitory molecules that render cells susceptible to exhaustion. It is a further object of the present invention to provide T cells which can be used in the treatment of cancer in a subject.

### Summary of the invention

These objects have been solved by the aspects of the present invention as specified hereinafter.

According to a first aspect of the invention, a method for stimulating T cells is provided, the method comprising incubating the T cells in a first medium comprising anti-TCRβ, anti-CD28 and IL-2, characterized in that the first medium additionally comprises IL-4 and TNFα.

In an embodiment of the first aspect, the first medium comprises IL-4 in a concentration of between 0.1 to 100 ng/ml, preferably between 0.5 to 50 ng/ml, more preferably between 3 to 20 ng/ml, even more preferably between 6 to 15 ng/ml, even more preferably between 9 to 12 ng/ml, most preferably 10 ng/ml; and TNFα in a concentration of between 1 to 1000 ng/ml, preferably between 10 to 500 ng/ml, more preferably between 30 to 200 ng/ml, even more preferably between 60 to 150 ng/ml, even more preferably between 90 to 120 ng/ml, most preferably 100 ng/ml.

In another embodiment of the first aspect, the first medium comprises anti-TCRβ in a concentration of between 0.05 to 20 µg/ml, preferably between 0.3 to 10 µg/ml, more preferably between 0.6 to 5 µg/ml, even more preferably between 0.9 to 2 µg/ml, even more preferably between 1 to 1.5 µg/ml, most preferably 1.25 µg/ml; anti-CD28 in a concentration of between 0.2 to 70 µg/ml, preferably between 1 to 50 µg/ml, more preferably between 2 to 25 µg/ml, even more preferably between 3 to 15 µg/ml, even more preferably 4 to 7 µg/ml, most preferably 5 µg/ml; and IL-2 in a concentration of between 1 to 200 U/ml, preferably between 5 to 100 U/ml, more preferably between 10 to 75 U/ml, even more preferably between 15 to 50 U/ml, even more preferably between 17 to 25 U/ml, most preferably 20 U/ml.

In an embodiment of the first aspect, the T cells are incubated in the first medium for at least 24 hours, preferably for at least 36 hours, more preferably for at least 48 hours, even more preferably for at least 60 hours, most preferably for at least 72 hours.

In a particular embodiment of the first aspect, the method comprises the steps of incubating naive T cells in a first medium comprising anti-TCRβ, anti-CD28, IL-2, IL-4, and TNFα, separating the T cells from the first medium, and incubating the T cells in a second medium comprising IL-2.

In a particular embodiment of the first aspect, the second medium comprises IL-2 in a concentration of between 1 to 200 U/ml, preferably between 5 to 100 U/ml, more preferably between 10 to 75 U/ml, even more preferably between 15 to 50 U/ml, even more preferably between 17 to 25 U/ml, most preferably 20 U/ml; and/or the T cells are incubated in the second medium for at least 24 hours, preferably for at least 36 hours, more preferably for at least 48 hours, even more preferably for at least 60 hours, most preferably for at least 72 hours.

In one embodiment of the first aspect, the T cell is a CD8⁺ T cell, most preferably a naive CD8⁺ T cell.

In another embodiment of the first aspect, the method is carried out ex *vivo.*

In an embodiment of the first aspect, the T cell is of human origin.

According to a second aspect of the invention, T cells are provided which are obtainable by the method according to the first aspect.

In an embodiment of the second aspect, the T cells are genetically modified to express chimeric antigen-specific T cell receptors (CAR T cell), preferably wherein the T cells are CAR T cells suitable for an allogenic immunotherapy treatment of pre-malignant or malignant cancer.

In another embodiment of the second aspect, after 30 days following intravenous injection of such T cells into the tail vein of a C57BL/6J mouse, at least 3%, preferably at least 7%, more preferably at least 11%, most preferably at least 15% of said T cells are present in the spleen of the C57BL/6J mouse.

In one embodiment of the second aspect of the present invention, determined on T cells present in the spleen of the mouse after 30 days following intravenous injection of the T cells into the tail vein of a C57BL/6J mouse, the amount of PD-1 expressed on the surface of the T cells is at least 16% less, preferably at least 32% less, more preferably at least 48% less, most preferably at least 64% less in comparison to the amount of PD-1 expressed on the surface of T cells incubated with IL-2 alone.

According to an embodiment of the second aspect of the present invention, and determined in accordance with the preceding embodiment, the amount of PD-1 expressed on the surface of the T cells is at least 5% less, preferably at least 10% less, more preferably at least 15% less, most preferably at least 20% less than the amount of PD-1 expressed on the surface of T cells incubated with IL-4.

According to one embodiment of the second aspect of the present invention, and determined in accordance with the preceding embodiments, the amount of TIM3 expressed on the surface of the T cells is at least 5% less, preferably at least 9% less, more preferably at least 14% less, most preferably at least 19% less than the amount of TIM3 expressed on the surface of T cells incubated with IL-2 alone.

According to an embodiment of the second aspect of the present invention, and determined in accordance with the preceding embodiments, the amount of TIM3 expressed on the surface of the T cells is at least 5% less, preferably at least 11% less, more preferably at least 16% less, most preferably at least 22% less than the amount of TIM3 expressed on the surface of T cells incubated with IL-4.

According to one embodiment of the second aspect of the present invention, and determined in accordance with the preceding embodiments, the amount of LAG3 expressed on the surface of the T cells is at least 7% less, preferably at least 14% less, more preferably at least 21% less, most preferably at least 28% less than the amount of LAG3 expressed on the surface of T cells incubated with IL-2 alone.

According to an embodiment of the second aspect of the present invention, and determined in accordance with the preceding embodiments, the amount of LAG3 expressed on the surface of the T cells is at least 5% less, preferably at least 10% less, more preferably at least 15% less, most preferably at least 20% less than the amount of LAG3 expressed on the surface of T cells incubated with IL-4.

According to a third aspect of the invention, a pharmaceutical composition is provided comprising the T cells according to the second aspect and a pharmaceutically acceptable excipient.

According to a fourth aspect of the invention, T cells according to the second aspect or the pharmaceutical composition according to the third aspect are provided for use as a medicament, or for use in a method of treating cancer in a subject, optionally wherein the cancer is a solid tumor, preferably malignant melanoma.

### Brief description of the drawings

The present disclosure will be more readily appreciated by reference to the following data when being considered in connection with the accompanying drawings in which:

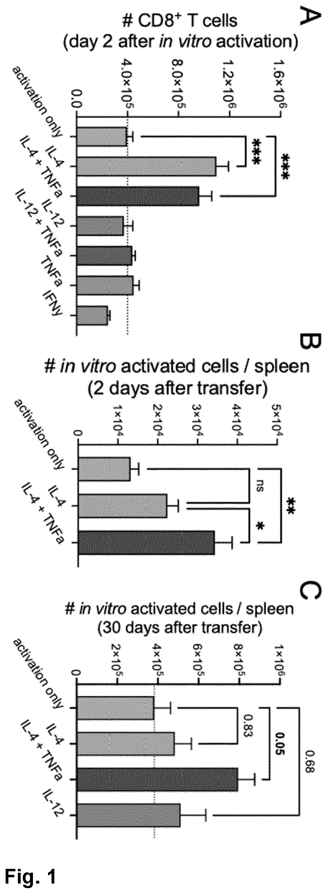

Figure 1 shows the effect of cytokines on the expansion and persistence of CD8⁺ T cells. Naive CD8⁺ T cells were activated *in vitro* with anti-TCRβ and anti-CD28 antibodies, and IL-2. Different cytokines and cytokine combinations were added to the medium during activation. On day 3 after activation, cells were washed and cultured for an additional 3 days without stimulation (resting phase). (A) Number of *in vitro* activated CD8⁺ T cells per well on day 2 after activation. (B) Number of *in vitro* activated CD8⁺ T cells transferred into recipient animals on day 6 after activation and recovered in the spleen on day 2 after transfer. (C) Number of *in vitro* activated CD8⁺ T cells transferred into recipient animals on day 6 after activation and recovered in the spleen at day 30 after transfer.

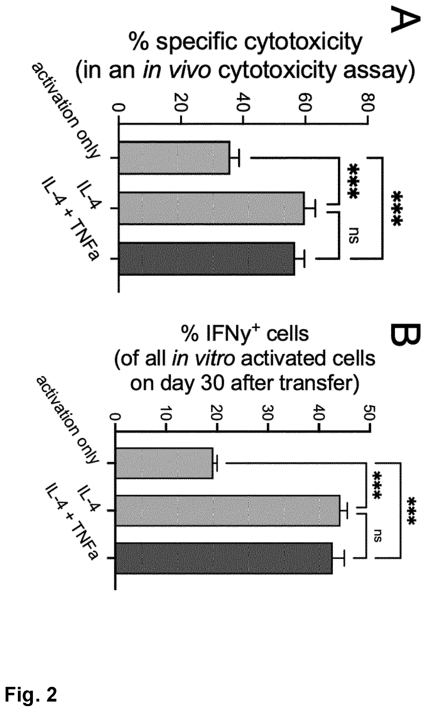

Figure 2 shows the effect of IL-4 and IL-4+TNFα on the effector function of CD8⁺ T cells. Naive OT-I T cells were activated *in vitro* with anti-TCRβ and anti-CD28 antibodies, and IL-2. During activation, different cytokines and cytokine combinations were added to the medium. On day 3 after activation, cells were washed and cultured for an additional 3 days without stimulation (resting phase). (A) *In vivo* cytotoxicity assay. For each condition, equal numbers of *in vitro* activated OT-I T cells were transferred into recipient animals on day 6 after activation. One day after transfer, a mixture of antigen-loaded target cells and unloaded control cells was injected, and 5 h later, the specific cytotoxicity was calculated from the ratio of target cells to control cells in the spleens of the experimental animals. (B) Proportion of IFNy-producing *in vitro* activated OT-I T cells recovered in the spleen 30 days after transfer into recipient animal and restimulated ex *vivo* for 4h with their specific antigen SIINFEKL (SEQ ID No. 1).

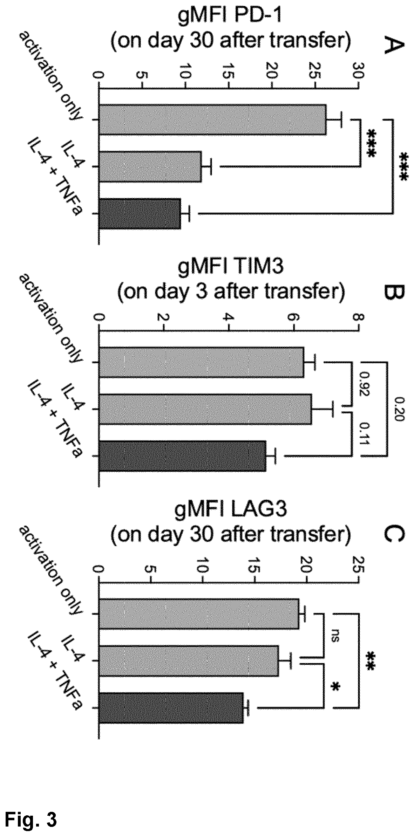

Figure 3 shows the effect of IL-4 and IL-4+TNFα on the expression of inhibitory molecules by CD8⁺ T cells, also known as T cell exhaustion markers. Naive CD8⁺ T cells were activated *in vitro* with anti-TCRβ and anti-CD28 antibodies, and IL-2. During activation, the indicated cytokines and cytokine combinations were added to the medium. On day 3 after activation, cells were washed and cultured for an additional 3 days without stimulation (resting phase). On day 6 after activation, the same number of cells per condition was transferred into recipient animals. On day 30 after transfer, the expression of the inhibitory molecules (A) PD-1, (B) TIM3, and (C) LAG3 was measured on the surface of the cells recovered in the spleens of recipient animals.

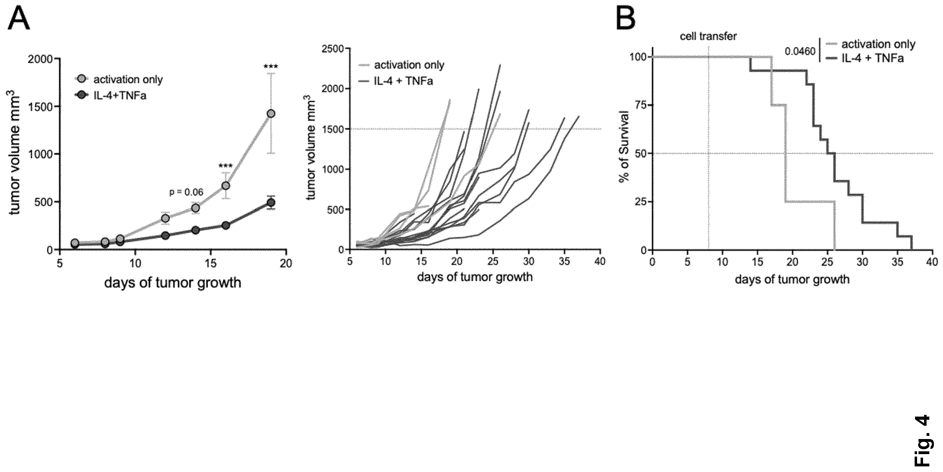

Figure 4 shows the effect of IL-4+TNFα on the anti-tumor response. 0.9*10⁶ OT-I T cells activated *in vitro* under the influence of IL-2 (activation only) or IL-2+IL-4+TNFα (IL-4+TNFα) with anti-TCRβ and anti-CD28 antibodies were transferred at day 6 after activation into recipient animals implanted with B16-OVA melanomas 8 days earlier. (A) Tumor growth. (B) Survival of the experimental animals.

These results will be interpreted in detail below.

### Detailed description of preferred embodiments

In the following, the invention will be explained in more detail with reference to the accompanying figures. In the Figures, like elements are denoted by identical reference numerals and repeated description thereof may be omitted in order to avoid redundancies.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

In order that the present description can be more readily understood, certain terms are first defined. Additional definitions are set forth throughout the detailed description.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International d'Unités (SI) accepted form. Numeric ranges are inclusive of the numbers defining the range. Unless otherwise indicated, nucleotide sequences are written left to right in 5' to 3' orientation. Amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower).

The term "lymphocyte" is used herein in the broadest sense to refer to a type of white blood cell found in the lymphatic system which are principal players in the adaptive immune response and the source of immune memory. Lymphocytes are broadly subdivided on the basis of functional and phenotypic differences into B lymphocytes (B cells), T lymphocytes (T cells), and innate lymphoid cells (ILCs).

The term "T lymphocyte" or "T cell" as used herein refers to a lymphocyte that expresses an antigen-binding T-cell receptor, which recognizes processed pieces of antigen bound to cell membrane proteins called major histocompatibility complex (MHC) molecules. T cells include T helper (T_{H}) cells and T cytotoxic (Tc) cells that are distinguished from one another by the presence of either CD4 (CD4⁺ T_{H} cells) or CD8 (CD8⁺ Tc cells) membrane glycoproteins on their surfaces.

The term "major histocompatibility complex molecules" or "MHC" as used herein refers to genetically diverse glycoproteins found on cell membranes which can form complexes with antigen and include MHC class I molecules, which are expressed by all nucleated cells of vertebrate species, and MHC class II molecules, which are expressed primarily by professional antigen-presenting cells (pAPCs) such as dendritic cells (DC), macrophages, and B cells. CD4⁺ T_{H} cells recognize antigen in complex with MHC class II and CD8⁺ Tc cells recognize antigen in complex with MHC class I.

The term "naive T cell" as used herein refers to a T cell which has not encountered its cognate antigen and acts as precursors for an effector and memory T cell (mature T cell). Naive T cells include T cells which express surface markers, such as CD45RA, CCR7, CD62L, CD127, and do not express markers of previous activation, such as CD25, CD44, CD69, or CD45RO.

The terms "chimeric antigen receptor", "chimeric T cell receptor", "artificial T cell receptor", or "CAR" are used interchangeably herein and refer in their broadest sense to a receptor that combines both antigen-binding and T cell activating functions into a single receptor. Generally, CARs comprise four domains: antigen recognition domain, hinge region, transmembrane domain, and intracellular T cell signaling domain. Approaches or sources used in CARs as antigen recognition domain comprise single chains of variable antibody fragments (ScFv) having light (VL) and heavy (VH) chains of immunoglobulins, TNF receptors, innate immune receptors, cytokines, structure proteins, and/or growth factors.

The term "antibody" is used herein in the broadest sense to refer to molecules with an immunoglobulin-like domain (for example IgG, IgM, IgA, IgD or IgE) and includes monoclonal, recombinant, polyclonal, chimeric, human, humanized, multispecific antibodies, including bispecific antibodies, and heteroconjugate antibodies; a single variable domain (e.g., VH, VHH, VL, domain antibody), antigen binding antibody fragments, Fab, F(ab')2, Fv, disulfide linked Fv, single chain Fv, disulfide-linked scFv, diabodies, etc. and modified versions of any of the foregoing.

By the terms "treat," "treating," or "treatment of" (or grammatically equivalent terms) it is meant that the severity of the subject's condition is reduced or at least partially improved or ameliorated and/or that some alleviation, mitigation or decrease in at least one clinical symptom is achieved and/or there is a delay in the progression of the condition.

As used herein, the terms "prevent," "prevents," or "prevention" and "inhibit," "inhibits," or "inhibition" (and grammatical equivalents thereof) are not meant to imply complete abolition of disease and encompasses any type of prophylactic treatment that reduces the incidence of the condition, delays the onset of the condition, and/or reduces the symptoms associated with the condition after onset.

An "effective," "prophylactically effective," or "therapeutically effective" amount as used herein is an amount that is sufficient to provide some improvement or benefit to the subject. Alternatively stated, an "effective," "prophylactically effective," or "therapeutically effective" amount is an amount that will provide some delay, alleviation, mitigation, or decrease in at least one clinical symptom in the subject. Those skilled in the art will appreciate that the effects need not be complete or curative, as long as some benefit is provided to the subject.

The term "subject" includes human and other mammalian subjects that receive either prophylactic or therapeutic treatment. As used herein, the term "subject" includes any human or non-human animal. The term "non-human animal" includes all vertebrates, e.g., mammals and non-mammals, such as non-human primates, sheep, dog, cow, chickens, amphibians, reptiles, etc.

As used herein, the terms "ug" and "uM" may be used interchangeably with "µg" and "µM," respectively.

As used herein, "administering" refers to the physical introduction of a composition comprising a therapeutic agent to a subject and encompasses any of the various methods and delivery systems known to those skilled in the art. Different routes of administration include intravenous, intraperitoneal, intramuscular, subcutaneous, spinal or other parenteral routes of administration, for example by injection or infusion. Parenteral administration encompasses modes of administration other than enteral and topical administration, usually by injection, and includes, without limitation, intravenous, intraperitoneal, intramuscular, intraarterial, intrathecal, intralymphatic, intralesional, intracapsular, intraorbital, intracardiac, intradermal, transtracheal, subcutaneous, subcuticular, intraarticular, subcapsular, subarachnoid, intraspinal, epidural and intrasternal injection and infusion, as well as *in vivo* electroporation.

Production methods for CAR T cells are well known in the art. Usually, they involve isolating T cells from a donor, stimulating (encompassing proliferation and expansion of the T cells), purifying and transducing them via a vector with a gene encoding the genetically modified CAR, and then administering them to the donor.

The methods of the disclosure are directed to stimulating T cells and comprise two phases. In the first phase, naive T cells are activated by anti-TCRβ and anti-CD28. In the context of the disclosure, this first phase is referred to as "activation phase". T cell activation refers to a process in which mature T cells can express antigen-specific T cell receptors on their surface to recognize their cognate antigens and respond by entering the cell cycle, secreting cytokines or lytic enzymes, and initiating the cell-based functions of the immune system.

In the second phase, the activating stimulus is removed while the T cells continue to expand. In the context of the disclosure, this second phase is referred to as "resting phase". The resting phase is intended to prevent the T cells from being overstimulated and consequently dying.

In both phases, the cell culture medium usually comprises IL-2 in order to support the expansion of the cells. IL-2 is a growth factor that supports the expansion of T cells and thus is used during the cultivation of T cells as a basic component of many cell culture mediums.

In the process for the isolation and differentiation of T cells, key properties of the activated T cells, such as tumor cell targeting, persistency, and efficacy, can be modulated by the addition of cytokines in the activation phase. Methods known in the art include the addition of cytokines such as IL-7, IL-12, IL-15, IL-18, and IL-21. Addition of these cytokines in the activation phase is known to increase the persistency of CAR-T cells. However, none of these have led to significant improvement in the efficacy of activated T cells in the treatment of solid tumors.

The present inventors have dedicated themselves to solving the problem of the present invention and were successful to find novel and useful methods for stimulating T cells having superior persistency and lower expression of inhibitory molecules. Specifically, the inventors succeeded in identifying the cytokine combination IL-4 and TNFα as a suitable stimulus to improve the expansion, persistency and functionality of CAR-T cells for the therapy of cancer in a subject, specifically solid tumors. The T cells activated in the presence of IL-4 and TNFα exhibit excellent expansion and cytotoxicity as well as increased persistency and a reduced expression of inhibitory molecules in comparison to combinations of cytokines known in the art.

The invention provides a method for stimulating T cells comprising incubating the T cells in a first medium comprising anti-TCRβ, anti-CD28 and IL-2, wherein the first medium additionally comprises IL-4 and TNFα.

In an embodiment of the invention, the first medium comprises IL-4 in a concentration of between 0.1 to 100 ng/ml, preferably between 0.5 to 50 ng/ml, more preferably between 3 to 20 ng/ml, even more preferably between 6 to 15 ng/ml, even more preferably between 9 to 12 ng/ml. In a particularly preferred embodiment of the invention, the first medium comprises IL-4 in a concentration of 10 ng/ml.

In a further embodiment, the first medium comprises TNFα in a concentration of between 1 to 1000 ng/ml, preferably between 10 to 500 ng/ml, more preferably between 30 to 200 ng/ml, even more preferably between 60 to 150 ng/ml, even more preferably between 90 to 120 ng/ml. In a particularly preferred embodiment of the invention, the first medium comprises TNFα in a concentration of 100 ng/ml.

In another embodiment, the first medium comprises anti-TCRβ in a concentration of between 0.05 to 20 µg/ml, preferably between 0.3 to 10 µg/ml, more preferably between 0.6 to 5 µg/ml, even more preferably between 0.9 to 2 µg/ml, even more preferably between 1 to 1.5 µg/ml, most preferably 1.25 µg/ml.

In a further embodiment, the first medium comprises anti-CD28 in a concentration of between 0.2 to 70 µg/ml, preferably between 1 to 50 µg/ml, more preferably between 2 to 25 µg/ml, even more preferably between 3 to 15 µg/ml, even more preferably 4 to 7 µg/ml, most preferably 5 µg/ml.

In another embodiment, the first medium comprises IL-2 in a concentration of between 1 to 200 U/ml, preferably between 5 to 100 U/ml, more preferably between 10 to 75 U/ml, even more preferably between 15 to 50 U/ml, even more preferably between 17 to 25 U/ml, most preferably 20 U/ml.

In an embodiment, the T cells are incubated in the first medium for at least 24 hours, preferably for at least 36 hours, more preferably for at least 48 hours, even more preferably for at least 60 hours, most preferably for at least 72 hours.

In another embodiment, the method comprises the steps of incubating naive T cells in a first medium comprising anti-TCRβ, anti-CD28, IL-2, IL-4, and TNFα, separating the T cells from the first medium, and incubating the T cells in a second medium comprising IL-2.

In another embodiment, the second medium comprises IL-2 in a concentration of between 1 to 200 U/ml, preferably between 5 to 100 U/ml, more preferably between 10 to 75 U/ml, even more preferably between 15 to 50 U/ml, even more preferably between 17 to 25 U/ml, most preferably 20 U/ml.

In yet another embodiment, the T cells are incubated in the second medium for at least 24 hours, preferably for at least 36 hours, more preferably for at least 48 hours, even more preferably for at least 60 hours, most preferably for at least 72 hours.

It is understood that the T cells may be incubated in both the first and second media for the same or different lengths of time.

According to a preferred embodiment, the first medium comprises IL-4 in a concentration of between 0.1 to 100 ng/ml, TNFα in a concentration of between 1 to 1000 ng/ml, anti-TCRβ in a concentration of between 0.05 to 20 µg/ml, and IL-2 in a concentration of between 1 to 200 U/ml, and the second medium comprises IL-2 in a concentration of between 1 to 200 U/ml, wherein the T cells are incubated in each of the first medium and the second medium for at least 24 hours.

Alternatively preferably, the first medium comprises IL-4 in a concentration of between 0.5 to 50 ng/ml, TNFα in a concentration of between 10 to 500 ng/ml, anti-TCRβ in a concentration of between 0.3 to 10 µg/ml, anti-CD28 in a concentration of between 0.2 to 70 µg/ml, and IL-2 in a concentration of between 5 to 100 U/ml, and the second medium comprises IL-2 in a concentration of between 5 to 100 U/ml, wherein the T cells are incubated in each of the first medium and the second medium for at least 36 hours.

Alternatively preferably, the first medium comprises IL-4 in a concentration of between 3 to 20 ng/ml, TNFα in a concentration of between 30 to 200 ng/ml, anti-TCRβ in a concentration of between 0.6 to 5 µg/ml, anti-CD28 in a concentration of between 1 to 50 µg/ml, and IL-2 in a concentration of between 10 to 75 U/ml, and the second medium comprises IL-2 in a concentration of between 10 to 75 U/ml, wherein the T cells are incubated in each of the first medium and the second medium for at least 48 hours.

Alternatively preferably, the first medium comprises IL-4 in a concentration of between 6 to 15 ng/ml, TNFα in a concentration of between 60 to 150 ng/ml, anti-TCRβ in a concentration of between 0.9 to 2 µg/ml, anti-CD28 in a concentration of between 2 to 25 µg/ml, anti-CD28 in a concentration of between 3 to 15 µg/ml, and IL-2 in a concentration of between 15 to 50 U/ml, and the second medium comprises IL-2 in a concentration of between 15 to 50 U/ml, wherein the T cells are incubated in each of the first medium and the second medium for at least 60 hours.

Alternatively preferably, the first medium comprises IL-4 in a concentration of between 9 to 12 ng/ml, TNFα in a concentration of between 90 to 120 ng/ml, anti-TCRβ in a concentration of between 1 to 1.5 µg/ml, anti-CD28 in a concentration of between 4 to 7 µg/ml, and IL-2 in a concentration of between 17 to 25 U/ml, and the second medium comprises IL-2 in a concentration of between 17 to 25 U/ml, wherein the T cells are incubated in each of the first medium and the second medium for at least 72 hours.

Alternatively preferably, the first medium comprises IL-4 in a concentration of 10 ng/ml, TNFα in a concentration of 100 ng/ml, anti-TCRβ in a concentration of 1.25 µg/ml, anti-CD28 in a concentration of 5 µg/ml, and IL-2 in a concentration of 20 U/ml, and the second medium comprises IL-2 in a concentration of 20 U/ml, wherein the T cells are incubated in each of the first medium and the second medium for at least 72 hours.

It is understood that it will be within the conventional knowledge of the skilled person to select an appropriate concentration of IL-4, TNFα, anti-TCRβ, anti-CD28, and IL-2 as well as appropriate incubation periods to increase the persistency and anti-tumor response of the activated effector cells.

Vessels in which naive T cells are incubated during the activation phase of the methods of the invention may be coated with anti-TCRβ and anti-CD28 antibodies. Alternatively, beads may be used on the surface of which these antibodies are bound (e.g., Dynabeads). The vessels may be coated with anti-TCRβ, and anti-CD28 may be added in soluble form, or *vice versa.*

As an alternative to the direct introduction of the cytokines IL-2, IL-4, and TNFα into the first and/or second medium as described herein, any of said cytokines may be introduced into the first medium and/or the second medium (IL-2) by other means known in the art. For instance, said cytokines may be introduced by the addition of dedicated cells into the medium capable of producing said cytokines. Said cytokines may also introduced by the addition of constructs to the medium capable of inducing production of said cytokines by the T cells themselves, for instance by transfection with mRNA or plasmids, viral transfection, etc. Alternatively, said cytokines may be introduced by the addition of dedicated agents into the medium capable of stimulating the corresponding cytokine receptors, e.g., stimulatory antibodies, synthetic cytokines, etc. Any method of introduction of the said cytokines is possible and is determined as per the discretion of the person skilled in the art on the basis of their general knowledge of the art.

In one embodiment, the T cell in the methods of the invention is a CD8⁺ T cell. In a preferred embodiment, the T cell is a naive CD8⁺ T cell. Methods for obtaining, isolating and culturing CD8⁺ cells from donors (e.g., human donors) are known in the art and comprise *inter alia* blood draw or bone marrow removal.

In another embodiment, the method is carried out ex *vivo.* In yet another embodiment, the T cell is of human origin.

The invention further provides T cells that are obtainable from the methods of the invention. Such T cells which may be obtained by executing the method of the present invention possess distinct and identifiably characteristics and are influenced by the method so that they can be distinguished from T cells disclosed in the prior art which are produced by different processes.

In an embodiment, the T cells are genetically modified to express chimeric antigen-specific T cell receptors (CARs). In a preferred embodiment, the T cells are CAR T cells suitable for an allogenic immunotherapy treatment of pre-malignant or malignant cancer. The cells may be autologous or non-autologous, preferably autologous.

In a further embodiment, after 30 days following intravenous injection of such T cells into the tail vein of a C57BL/6J mouse, at least 3%, preferably at least 7%, more preferably at least 11%, most preferably at least 15% of said T cells are present in the spleen of the C57BL/6J mouse. The presence and the cell number of the transferred T cells may be assessed by identifying their specific T cell receptor by methods such as (but not limited to) staining with antigen-loaded MHC class I multimers (Chang J. MHC multimer: A Molecular Toolbox for Immunologists. Mol Cells. 2021 May 31 ;44(5):328-334., Alanio C., et al. "Tracking antigen-specific CD8+ T cells using MHC class I multimers." Antigen Processing: Methods and Protocols (2013): 309-326.) or TCR sequencing (De Simone, M., et al.. "Single cell T cell receptor sequencing: techniques and future challenges." Frontiers in Immunology 9 (2018): 1638.) or CD107a surface expression (Betts, M. R., et al. "Sensitive and viable identification of antigen-specific CD8+ T cells by a flow cytometric assay for degranulation." Journal of immunological methods 281.1-2 (2003): 65-78.) or IFNy production after stimulation with the specific antigen (Ghanekar, S. A., et al. "Gamma interferon expression in CD8+ T cells is a marker for circulating cytotoxic T lymphocytes that recognize an HLA A2-restricted epitope of human cytomegalovirus phosphoprotein pp65." Clinical Diagnostic Laboratory Immunology 8.3 (2001): 628-631.).

In one particular embodiment, determined on T cells present in the spleen of the mouse after 30 days following intravenous injection of the T cells into the tail vein of a C57BL/6J mouse, the amount of PD-1 expressed on the surface of the T cells is at least 16% less, preferably at least 32% less, more preferably at least 48% less, most preferably at least 64% less than the amount of PD-1 expressed on the surface of T cells incubated with IL-2 alone (activation only), and at least 5% less, preferably at least 10% less, more preferably at least 15% less, most preferably at least 20% less than the amount of PD-1 expressed on the surface of T cells incubated with IL-4.

In another particular embodiment, determined on T cells present in the spleen of the mouse after 30 days following intravenous injection of the T cells into the tail vein of a C57BL/6J mouse, the amount of TIM3 expressed on the surface of the T cells is at least 5% less, preferably at least 9% less, more preferably at least 14% less, most preferably at least 19% less than the amount of TIM3 expressed on the surface of T cells incubated with IL-2 alone (activation only), and at least 5% less, preferably at least 11% less, more preferably at least 16% less, most preferably at least 22% less than the amount of TIM3 expressed on the surface of T cells incubated with IL-4.

In another particular embodiment, determined on T cells present in the spleen of the mouse after 30 days following intravenous injection of the T cells into the tail vein of a C57BL/6J mouse, the amount of LAG3 expressed on the surface of the T cells is at least 7% less, preferably at least 14% less, more preferably at least 21% less, most preferably at least 28% less than the amount of LAG3 expressed on the surface of T cells incubated with IL-2 alone (activation only), and at least 5% less, preferably at least 10% less, more preferably at least 15% less, most preferably at least 20% less than the amount of LAG3 expressed on the surface of T cells incubated with IL-4.

In the methods of the present invention, recombinant human IL-2 (SEQ ID No. 2) may be used as IL-2. Also preferably, recombinant human IL-4 (SEQ ID No. 3) may be used as IL-4, Further, TNFα may preferably be recombinant human TNFα (SEQ ID No. 4). Preferably, IFNy may be recombinant human IFNy (SEQ ID No. 5). IL-12 may preferably be composed of recombinant human IL-12 p35 subunit (SEQ ID No. 6) and the recombinant human IL-12 p40 subunit (SEQ ID No. 7).

Alternatively, in the methods of the invention, IL-4 may preferably be recombinant murine IL-4 (SEQ ID No. 8). TNFα may also preferably be recombinant murine TNFα (SEQ ID No. 9). IFNy as used herein may preferably be recombinant murine IFNy (SEQ ID No. 10). IL-12 may alternatively preferably be composed of recombinant murine IL-12 p35 subunit (SEQ ID No. 11) and the recombinant murine IL-12 p40 subunit (SEQ ID No. 12).

Furthermore, the anti-mouse TCRβ antibody as used in the context of the present invention may preferably be the anti-mouse TCRβ antibody clone H57-597 (Catalogue/Order # 109202; BioLegend Inc., San Diego, CA). The anti-mouse CD28 antibody as used in the context of the present invention may preferably be the anti-mouse CD28 antibody clone 37.51 (Catalogue/Order # 102116; BioLegend Inc., San Diego, CA).

The anti-human TCRβ antibody as used in the context of the present invention may preferably be the anti-human TCRβ antibody clone WT31 (Catalogue/Order #347770; BD Biosciences, Franklin Lakes, NJ). The anti-human CD3 antibody as used in the context of the present invention may preferably be the anti-human CD3 antibody clone UCHT1 (Catalogue/Order #555329; BD Biosciences, Franklin Lakes, NJ). The anti-human CD28 antibody as used in the context of the present invention may preferably be the anti-human CD28 antibody clone CD28.2 (Catalogue/Order #555725; BD Biosciences, Franklin Lakes, NJ). The invention further provides pharmaceutical compositions comprising the T cells of the invention and a pharmaceutically acceptable excipient.

The invention further provides the T cells or the pharmaceutical compositions of the invention for use as a medicament.

The invention further provides the T cells or the pharmaceutical compositions of the invention for use in a method of treating cancer in a subject. The T cells or the pharmaceutical compositions of the invention may be administered to the subject by ways known in the art, preferably by intravenous infusion. The subject is preferably a human patient, preferably the donor of the cells.

The T cells or pharmaceutical compositions of the invention may be used in combination with immunosuppressive treatment and/or one or more other therapies against cancer selected from the non-exhaustive list comprising antibody therapy, chemotherapy, cytokine therapy, dendritic cell therapy, gene therapy, hormone therapy, laser light therapy, and radiation therapy. The subject may be treated with immunosuppressive treatment and/or one or more other therapies against cancer prior to, after, and/or concurrently to the treatment with the T cells or pharmaceutical compositions of the invention.

In a particular embodiment, the cancer is a solid tumor, preferably malignant melanoma. The cancer may be adult tumors/cancers and pediatric tumors/cancers. Other and additional diseases and disorders which may be treated using the present invention are apparent to the skilled person and comprise particularly (but not limited to) different types of cancer such as lung cancer, gastric cancer, renal cell cancer, colon cancer, breast cancer, ovarian cancer, urothelial cancer, pancreatic cancer, myeloma, Hodgkin's lymphoma, retinoblastoma, leukemia, cervical cancer, esophageal cancer, glioma, non-Hodgkin's lymphoma, hepatocellular cancer, oral cancer, and others. For instance, the cancer may be selected from the non-exhaustive list comprising acute myeloid leukemia (AML), breast carcinoma, cholangiocarcinoma, colorectal adenocarcinoma, extrahepatic bile duct adenocarcinoma, female genital tract malignancy, gastric adenocarcinoma, gastroesophageal adenocarcinoma, gastrointestinal stromal tumors (GIST), glioblastoma, head and neck squamous carcinoma, leukemia, liver hepatocellular carcinoma, low grade glioma, lung bronchoalveolar carcinoma (BAC), lung non-small cell lung cancer (NSCLC), lung small cell cancer (SCLC), lymphoma, male genital tract malignancy, malignant solitary fibrous tumor of the pleura (MSFT), multiple myeloma, neuroendocrine tumor, nodal diffuse large B-cell lymphoma, non-epithelial ovarian cancer (non-EOC), ovarian surface epithelial carcinoma, pancreatic adenocarcinoma, pituitary carcinomas, oligodendroglioma, prostatic adenocarcinoma, retroperitoneal or peritoneal carcinoma, retroperitoneal or peritoneal sarcoma, small intestinal malignancy, soft tissue tumor, thymic carcinoma, thyroid carcinoma, uveal melanoma, or any combination thereof.

All embodiments of the present invention as disclosed and described herein are deemed to be combinable in any combination, unless the skilled person considers such a combination to not make any technical sense. The invention is now further explained by individual examples which are intended to illustrate but not to limit the present invention.

### EXAMPLES

### Expansion and Persistence

For an analysis of the effect of cytokines on the expansion and persistence of CD8⁺ T cells, naive CD8⁺ T cells were activated *in vitro* with anti-TCRβ and anti-CD28 antibodies. Different cytokines and cytokine combinations were added to the medium during activation. On day 3 after activation, the cells were washed and cultured for an additional 3 days without stimulation (resting phase). Fig. 1A shows the number *of in vitro* activated CD8⁺ T cells per well at day 2 after activation. Fig. 1B shows the number of *in vitro* activated CD8⁺ T cells transferred into recipient animals at day 6 after activation and recovered in the spleen at day 2 after transfer. Fig. 1C shows the number of *in vitro* activated CD8⁺ T cells transferred into recipient animals at day 6 after activation and recovered in the spleen at day 30 after transfer.

The present inventors found that naive CD8⁺ T cells activated *in vitro* with anti-TCRβ and anti-CD28 antibodies show significantly improved expansion after stimulation with IL-4 or IL-4+TNFα than after stimulation with IL-12, IL-12+TNFα, IFNy, or TNFα (see Fig. 1A). However, while *in vitro* expansion of IL-4 and IL-4+TNFα-stimulated T cells is comparable, IL-4+TNFα-stimulated T cells exhibit significantly higher persistence *in vivo* than IL-4-stimulated cells after transfer into recipient animals (see Fig. 1B and 1C).

This result shows that while both the stimulation according to the present invention as well as stimulation with IL-4 alone improve the activation and expansion of T cells, only the stimulation according to the present invention is able to increase T cell persistence beyond the activation phase.

### Effector Function

In this experiment, the effect of IL-4 and IL-4+TNFα on the effector function of CD8⁺ T cells is analyzed. Naive OT-I T cells were activated *in vitro* with anti-TCRβ and anti-CD28 antibodies. During activation, different cytokines and cytokine combinations were added to the medium. On day 3 after activation, the cells were washed and cultured for an additional 3 days without stimulation (resting phase).

Fig. 2A shows the results of an *in vivo* cytotoxicity assay, in which for each condition equal numbers of *in vitro* activated OT-I T cells were transferred into recipient animals on day 6 after activation. One day after the transfer, a mixture of antigen-loaded target cells and unloaded control cells was injected, and 5 h later, the specific cytotoxicity was calculated from the ratio of target cells to control cells in the spleens of the experimental animals.

Fig. 2B shows proportions of IFNy-producing *in vitro* activated OT-I T cells recovered in the spleen 30 days after transfer to recipient animal and restimulated ex *vivo* for 4h with SIINFEKL.

The present inventors found that stimulation of CD8⁺ T cells with IL-4 and IL-4+TNFα during their activation not only increases cell expansion and persistence but also significantly enhances the cytotoxicity of the resulting effector cells compared with just IL-2 (activation only) (cf. Fig. 2A). The enhanced cytotoxicity by stimulation with IL-4 and IL-4+TNFα persists after transfer of the cells into recipient animals (cf. Fig. 2A), and even 30 days after their transfer, more IL-4- and IL-4+TNFα-stimulated T cells than IL-2-stimulated cells (activation only) produce effector molecules such as IFNy (cf. Fig. 2C). The addition of TNFα did not negate or limit the effect of IL-4.

Taken together, these findings indicate that stimulation according to the present invention improves the cytotoxicity of T cells, albeit not beyond treatment with one of its components, IL-4 alone.

### Inhibitory Molecules

The effect of IL-4 and IL-4+TNFα on the expression of inhibitory molecules was assessed by activating naive CD8⁺ T cells *in vitro* with anti-TCRβ and anti-CD28 antibodies. During activation, the indicated cytokines and cytokine combinations were added to the medium. On day 3 after activation, cells were washed and cultured for an additional 3 days without stimulation (resting phase). On day 6 after activation, the same number of cells per condition was transferred into recipient animals. On day 30 after transfer, the expression of the inhibitory molecules PD-1 (Fig. 3A), TIM3 (Fig. 3B), and LAG3 (Fig. 3C) was measured on the surface of the cells recovered in the spleens of recipient animals.

The immune response in the tumor microenvironment can be throttled by stimulating inhibitory molecules such as PD-1, TIM3, and LAG3 on the surface of T cells. The present inventors found that CD8⁺ T cells stimulated with IL-4+TNFα during their activation expressed significantly less inhibitory molecules such as PD-1 (cf. Fig. 3A) and LAG3 (cf. Fig. 3C) and tended to express less TIM3 (cf. Fig. 3B) than IL-2-stimulated cells 30 days after transfer into recipient animals. In contrast, CD8⁺ T cells stimulated with IL-4 only expressed less PD-1 (cf. Fig. 3A).

These findings indicate that stimulation according to the present invention reduces exhaustion of T cells and thereby also susceptibility to immune escape strategies employed by tumors. Most importantly, the differential expression of cell surface molecules between cells treated according to the present invention or according to the state of the art allows discrimination between cells depending on how they were stimulated.

### Anti-Tumor Response in the B16 Tumor Model

The effect of IL-4+TNFα on the anti-tumor response was analyzed by activating OT-I T cells *in vitro* under the influence of IL-2 (activation only) or IL-2+IL-4+TNFα (IL-4+TNFα) with anti-TCRβ and anti-CD28 antibodies and transferring 0.9*10⁶ of these cells at day 6 after activation into recipient animals implanted with B16-OVA melanomas 8 days earlier. Fig. 4A illustrates the tumor growth over time and Fig. 4B the survival of the experimental animals over time.

The present inventors found that OT-I T cells activated under the influence of IL-4+TNFα, after their transfer into recipient animals bearing already established B16-OVA melanomas, are able to significantly delay tumor growth (cf. Fig. 4A) and prolong survival of the experimental animals (cf. Fig. 4B).

These findings indicate that the present invention is able to cause delayed tumor growth and prolonged survival in a mouse model.

### Discussion

In the mouse model, the present inventors discovered that stimulation of T cells with the cytokine combination IL-4+TNFα significantly increases the efficiency of the generated effector cells. IL-4+TNFα-stimulated CD8⁺ T cells show enhanced expansion (cf. Fig. 1A), increased persistency (cf. Fig. 1B and 1C), increased cytotoxicity (cf. Fig. 2), decreased expression of inhibitory molecules (cf. Fig. 3), and improved anti-tumor response (cf. Fig. 4). Several cytokine combinations were tested, some of which are shown in Fig. 1, wherein IL-4+TNFα was identified as the most effective combination.

While IL-4 alone may increase the expansion and cytotoxicity of CD8⁺ T cells, only in combination with TNFα was an improvement in persistency and a reduction in the expression of inhibitory molecules induced. IL-13 has a similar but weaker effect than IL-4. Because the persistency of CAR T cells is important for the effective treatment of cancer, the observed additive effect of TNFα to stimulation with IL-4 alone represents an important and unexpected improvement. The reduced expression of inhibitory molecules also makes IL-4+TNFα-stimulated cells less susceptible to suppression in the tumor microenvironment.

B16 melanoma model is a tumor model with very low immunogenicity. The slowing of tumor growth and prolongation of survival of the experimental animals by IL-4+TNFα-stimulated OT-I cells are therefore a significant therapeutic success.

These findings show that the efficiency of CAR-T cell therapy can be increased if the CAR-T cells are treated beforehand by adding the cytokine combination of the present invention. Not only are the T cells more effective, but they also expand and survive better, so that fewer CAR-T cells need to be transferred to achieve efficacy. This procedure is applicable to all CAR-T cell therapies because it is not antigen-specific. It does not require additional treatments of CAR-T cells, as cytokines are simply added during standard activation.

### METHODS

### Isolation and differentiation of CD8⁺ T cells

Naive CD8⁺ T cells were isolated from the spleens of C57BL/6J or OT-I mice by depletion of Ter119', B220⁺, CD11b⁺, CD4⁺ and CD44^{high} cells using MACS (magnetic activated cell sorting). For activation of the cells, F-bottom 96 well plates were coated with 1.25 µg/ml anti-mouse TCRβ and 5 µg/ml anti-mouse CD28 for 3 h at 37 °C, and then washed twice with PBS and once with RPMI1640 medium. Per well, 1*10⁵ naive T cells were activated in 200 µl complete medium (RPMI1640 medium containing 10% FCS, 500 µM β-mercaptoethanol) with 20 U/ml rhlL-2. When indicated, 10 ng/ml IL-4, 10 ng/ml IL-12, 10 ng/ml IFNγ and/or 100 ng/ml TNFα were added to the medium. At 3 days after activation, cells were collected, washed, and cultured for a 3-day rest period in complete medium containing 20 U/ml rhlL-2 without additional stimulation. On day 6 after activation, T cells were collected again, washed, counted, and used for different assays.

### Transfer of in vitro activated T cells into recipient animals (non-tumor-bearing animals).

3*10⁶ *in vitro* activated CD8⁺ T cells were thoroughly washed with PBS and transferred in 100 µl PBS by i.v. injection into the tail vein of the recipient animals. At the indicated time points, the transferred cells were then analyzed in the spleens of the recipient animals.

### B16-OVA tumor model

B16-OVA tumor cells were cultured at 80% confluence in RPMI1640 containing 10% FCS and 417 µg/ml G418. After one week in culture, 10⁶ tumor cells were implanted into the flank of shaved mice in 100 µl PBS by s.c. injection. Eight days after tumor implantation, 0.9*10⁶ *in vitro* activated OT-I T cells were then transferred by i.v. injection into the tail vein. Tumor growth was measured three times a week and calculated as follows: [Tumor volume] = 0.5 * [tumor length] * [tumor width]²

## Claims

1. A method for stimulating T cells, the method comprising incubating the T cells in a first medium comprising anti-TCRβ, anti-CD28 and IL-2; **characterized in that**
the first medium additionally comprises IL-4 and TNFα.

2. The method of claim 1, wherein the first medium comprises
(i) IL-4 in a concentration of between 0.1 to 100 ng/ml, preferably between 0.5 to 50 ng/ml, more preferably between 3 to 20 ng/ml, even more preferably between 6 to 15 ng/ml, even more preferably between 9 to 12 ng/ml, most preferably 10 ng/ml; and
(ii) TNFα in a concentration of between 1 to 1000 ng/ml, preferably between 10 to 500 ng/ml, more preferably between 30 to 200 ng/ml, even more preferably between 60 to 150 ng/ml, even more preferably between 90 to 120 ng/ml, most preferably 100 ng/ml.

3. The method of claim 1 or 2, wherein the first medium comprises
(iii) anti-TCRβ in a concentration of between 0.05 to 20 µg/ml, preferably between 0.3 to 10 µg/ml, more preferably between 0.6 to 5 µg/ml, even more preferably between 0.9 to 2 µg/ml, even more preferably between 1 to 1.5 µg/ml, most preferably 1.25 µg/ml;
(iv) anti-CD28 in a concentration of between 0.2 to 70 µg/ml, preferably between 1 to 50 µg/ml, more preferably between 2 to 25 µg/ml, even more preferably between 3 to 15 µg/ml, even more preferably 4 to 7 µg/ml, most preferably 5 µg/ml; and
(v) IL-2 in a concentration of between 1 to 200 U/ml, preferably between 5 to 100 U/ml, more preferably between 10 to 75 U/ml, even more preferably between 15 to 50 U/ml, even more preferably between 17 to 25 U/ml, most preferably 20 U/ml.

4. The method of any of claims 1 to 3, wherein the T cells are incubated in the first medium for at least 24 hours, preferably for at least 36 hours, more preferably for at least 48 hours, even more preferably for at least 60 hours, most preferably for at least 72 hours.

5. The method of any of claims 1 to 4, comprising the steps of:
(i) Incubating naive T cells in a first medium comprising anti-TCRβ, anti-CD28, IL-2, IL-4, and TNFα;
(ii) Separating the T cells from the first medium;
(iii) Incubating the T cells in a second medium comprising IL-2.

6. The method of claim 5, wherein
(i) the second medium comprises IL-2 in a concentration of between 1 to 200 U/ml, preferably between 5 to 100 U/ml, more preferably between 10 to 75 U/ml, even more preferably between 15 to 50 U/ml, even more preferably between 17 to 25 U/ml, most preferably 20 U/ml; and/or
(ii) the T cells are incubated in the second medium for at least 24 hours, preferably for at least 36 hours, more preferably for at least 48 hours, even more preferably for at least 60 hours, most preferably for at least 72 hours.

7. The method of any of claims 1 to 6, wherein the T cell is a CD8⁺ T cell, most preferably a naive CD8⁺ T cell.

8. The method of any of claims 1 to 7, wherein the method is carried out ex *vivo.*

9. The method of any of claims 1 to 8, wherein the T cell is of human origin.

10. T cells obtainable by the method of any of claims 1 to 9.

11. The T cells of claim 10, wherein the T cells are genetically modified to express chimeric antigen-specific T cell receptors (CAR), preferably wherein the T cells are CAR T cells suitable for an allogenic immunotherapy treatment of pre-malignant or malignant cancer.

12. The T cells of claim 11 or 12, wherein after 30 days following intravenous injection of such T cells into the tail vein of a C57BL/6J mouse, at least 3%, preferably at least 7%, more preferably at least 11%, most preferably at least 15% of said T cells are present in the spleen of the C57BL/6J mouse.

13. The T cells of any of claims 10 to 12, wherein, determined on T cells present in the spleen of the mouse after 30 days following intravenous injection of the T cells into the tail vein of a C57BL/6J mouse,
(i) the amount of PD-1 expressed on the surface of the T cells is at least 16%, preferably at least 32%, more preferably at least 48%, most preferably at least 64% less than the amount of PD-1 expressed on the surface of T cells incubated with IL-2 alone (activation only), and at least 5%, preferably at least 10%, more preferably at least 15%, most preferably at least 20% less than the amount of PD-1 expressed on the surface of T cells incubated with IL-4; and/or
(ii) the amount of TIM3 expressed on the surface of the T cells is at least 5%, preferably at least 9%, more preferably at least 14%, most preferably at least 19% less than the amount of TIM3 expressed on the surface of T cells incubated with IL-2 alone (activation only), and at least 5%, preferably at least 11%, more preferably at least 16%, most preferably at least 22% less than the amount of TIM3 expressed on the surface of T cells incubated with IL-4; and/or
(iii) the amount of LAG3 expressed on the surface of the T cells is at least 7%, preferably at least 14%, more preferably at least 21%, most preferably at least 28% less than the amount of LAG3 expressed on the surface of T cells incubated with IL-2 alone (activation only), and at least 5%, preferably at least 10%, more preferably at least 15%, most preferably at least 20% less than the amount of LAG3 expressed on the surface of T cells incubated with IL-4.

14. A pharmaceutical composition comprising the T cells of any one of claims 11 to 13 and a pharmaceutically acceptable excipient.

15. The T cells of claims 11 to 13 or the pharmaceutical composition of claim 14 for use as a medicament, or for use in a method of treating cancer in a subject, optionally wherein the cancer is a solid tumor, preferably malignant melanoma.
